# EUROPEAN PATENT SPECIFICATION

(11) **EP 2 267 161 B1**
(45) Date of publication and mention of the grant of the patent: **16.07.2014**
(21) Application number: 10173977.9
(22) Date of filing: 15.06.2006
(51) Int. Cl.: C12Q 1/68

(54) **Primers, probes and kits for the detection of bacterial species belonging to the genus bartonella**
Nukleinsäureprimer, Sonden und Kits für den Nachweis von Bakterien der Gattung Bartonella
Amorces des acides nucléiques, sondes et kits pour la détection des bactéries du genre Bartonella

(30) Priority: 17.06.2005 ES 200501481; 17.06.2005 US 691231 P
(43) Date of publication of application: 29.12.2010
(62) Divisional of application: 06764381.7
(73) Proprietor: Instituto de Salud Carlos III, 28029 Madrid (ES)
(72) Inventor: Anda Fernandez, Pedro, 28029, Madrid (ES); Escudero Nieto, Raquel, 28029, Madrid (ES); Jado Garcia, Isabel, 28029, Madrid (ES); Rodriquez Moreno, Isabel, 28029, Madrid (ES); Jimenez Alonso, María Isabel, 28029, Madrid (ES)
(74) Representative: Pons Ariño, Angel

(56) References cited:
- US-B1- 6 300 072
- LI DONG-MEI ET AL: "Study on Bartonella infection using molecular biological diagnostic techniques from China" ZHONGHUA LIU XING BING XUE ZA ZHI = ZHONGHUA LIUXINGBINGXUE ZAZHI,, vol. 25, no. 7, 1 July 2004 (2004-07-01), pages 602-606, XP009140145
- ROUX V ET AL: "INTER- AND INTRASPECIES IDENTIFICATION OF BARTONELLA (ROCHALIMAEA) SPECIES" JOURNAL OF CLINICAL MICROBIOLOGY, AMERICAN SOCIETY FOR MICROBIOLOGY, WASHINGTON, DC, US, vol. 33, 1 June 1995 (1995-06-01), pages 1573-1579, XP002926816 ISSN: 0095-1137
- MAGGI RICARDO G ET AL: "Potential limitations of the 16S-23S rRNA intergenic region for molecular detection of Bartonella species." JOURNAL OF CLINICAL MICROBIOLOGY MAR 2005 LNKD- PUBMED:15750079, vol. 43, no. 3, March 2005 (2005-03), pages 1171-1176, XP002606098 ISSN: 0095-1137
- DILLON B ET AL: "Limited diversity among human isolates of Bartonella henselae." JOURNAL OF CLINICAL MICROBIOLOGY DEC 2002 LNKD- PUBMED:12454174, vol. 40, no. 12, December 2002 (2002-12), pages 4691-4699, XP002606099 ISSN: 0095-1137
- MINNICK MICHAEL F ET AL: "Identification of Bartonella using PCR: Genus- and species-specific primer sets" JOURNAL OF MICROBIOLOGICAL METHODS, vol. 31, no. 1-2, December 1997 (1997-12), pages 51-57, XP002606100 ISSN: 0167-7012

## Description

The present invention relates to the detection and identification of bacterial speciesthat cause zoonosis, based on DNA analysis. More specifically, the invention provides primers and kit for the detection of bacteria belonging to the genera *Bartonella* based on DNA amplification.

### BACKGROUND

To date, 200 zoonotic diseases (bartonelosis, leptospirosis, Lyme borreliosis...) which affect humans have been described. In third world countries, they represent one of the main causes of death and entail substantial economic loss. Coexistence with animals, lack of sanitary infrastructure and low cultural level continue to be the main allies of these diseases.

Certain types of zoonosis are now thriving in industrialized countries as a consequence of population increases in urban and periurban areas, in addition to increased movement of animals across international borders, which entails the risk of introducing exotic diseases into our environment.

These circumstances, coupled with the frequent findings of arthropods infected by more than one of the pathogens included in the present invention, increase the possibility of being transmitted more than one of the bacterial species included in the present invention in a single sting.

As a result, hospitalizations due to medical profiles produced by human contact with animals or different classes of arthropods, such as mosquitoes, ticks, fleas, lice, mites, etc., which act as vectors or pathogen reservoirs, is becoming increasingly common. Said medical profiles, due to their high degree of similarity, do not allow a fast and reliable identification of the pathogenous agent, so that specific and fast treatment is not possible and is occasionally administered too late. This undoubtedly justifies the need for a comprehensive detection method.

The diagnostic methods currently available are limited to detecting antibodies which, in general, is retrospective and of little use to treating patients in acute-phase states. Culture is not considered a diagnostic method, due both to its technological complexity, which excludes it from regular practice in hospital microbiology laboratories, and to the need for P3 facilities.

Molecular diagnosis by genome amplification by means of PCR represents a diagnostic option of great value. However, clinical samples of sufficient quantity for pathogen testing or the methodology required to carry out different tests are not always available.

LI DONG-MEI ET AL: "Study on Bartonella infection using molecular biological diagnostic techniques from China" ZHONGHUA LiU XING BING XUE ZA ZHI = ZHONGHUA LIUXINGBINGXUE ZAZHI" vol. 25, no. 7, 1 July 2004 (2004-07-01), pages 602-606 discloses a pair of primers, each primer binds to a conserved tRNA region in the 16S - 23S spacer. The region between the tRNAs (i.e. the region amplified by the primers) is said to be highly variable and different species of Bartonella are distinguished on the basis of the amplicon produced (see Tables on pages 604 and 605). The primer pair Tlle.455p and TAla.885n bind at 464-485 and 894-914 of the reference sequence, respectively. The different Bartonella species amplified are B.alsatica, B.bacilliformis, B.elizabethae, B.vinsonii vinsonii, B.vinsonii berkhofii, B.vinsonii arupensis, B.tribocorum, B.taylorii, *B.quintana, B.koehlerae, B*.*henselae* and *B.grahamii.*

A paper has recently been published (Blaskovic D. et al. 2005. Oligo-based detection of tick-borne bacteria. FEMS Microbiology Letters 243:273-8) which describes a method for the detection of 5 out of 6 pathogens proposed by the present invention. Said method is based on ribosomal DNA analysis and uses universal primers, which amplify the genetic material of both target and non-target bacteria, due to which its sensitivity is substantially reduced.

Other methods, such as those described by American patents US 6,300,072 and 6,518,020, are capable of detecting and identifying bacteria of the genus ***Bartonella,*** by using the same DNA region (intergenic space 16S-23S). However, the number of species within this genus has increased substantially since said patents were filed and their approximation, which consists of discriminating between species according to the size of the amplicon obtained during PCR, is not useful for certain known species within the same genus which are similar in size to the amplified fragment.

While the method provided by the present invention also proposes using intergenic region 16S-23S for the detection of species belonging to the genus ***Bartonella,*** improvements have been introduced with respect to the previously described procedures, as it is capable of detecting a much wider range of species within the same and other genera, using completely new probes and primers with maximum sensitivity levels.

For the detection of ***Coxiella burnetii,*** the same primers and DNA region (insertion sequence IS1111) as the previously described methods are used. Said detection has been improved by combining it with another series of completely new tests aimed at identifying other bacterial species, which can be transmitted by the same vectors and also provide a new hybridization probe for the detection of ***Coxiella burnetii.***

### DESCRIPTION OF THE INVENTION

Definitions: Multiple PCR or Multiplex PCR: PCR (Polymerase Chain Reaction) is a system whereby the number of copies of a specific nucleotide sequence of an organism is amplified or increased using two primers. Multiple PCR or Mutiplex PCR is a variation of PCR which allows the simultaneous amplification of more than one target sequence using more than one pair of primers.

The present invention solves the problem of the tediousness and complexity of detecting a high number of bacteria that cause zoonosis which can be clinically and/or epidemiologically indistinguishable, through the development of a method and Kit for the detection of bacterial species that cause zoonosis belonging to the genera ***Bartonella.***

The solution found by the present invention consists of analyzing a bacterial DNA region to determine which species are present. Specifically, the **intergenic space 16S-23S** is analyzed to detect the presence of ***Bartonella.***

According to the above, it is disclosed a method for the sample-based detection of bacteria, comprised of the following steps:
i) Placing the sample under analysis in contact with a reaction mixture containing specific primers to carry out Multiplex PCR.
ii) Amplifying by means of polymerase chain reaction.
iii) Identifying the formation of the products in the previous step, said information being indicative of the presence or absence of zoonosis-causing bacteria.

In relation to this, it is disclosed a method to detect:
- *Bartonella henselae, B. quintana, B. clarridgeiae, B. elizabethae, B. grahamii, B. vinsonii subspecies berkhofii, B. vinsonii* • *subspecies vinsonii, B. vnisonii subspecies aurupensis, B. bacilliformis, B. alsatica, B. bovis, B. doshiae, B. koehlerae, B. schoenbuchensis, B. taylori* and *B. tribocorum,*
all of which are capable of causing zoonosis, jointly infecting an individual and being difficult to identify by simple observation of medical profiles, based on the amplification and analysis of the genes or specific genic regions presented in table 2.

According to a specific embodiment of this first aspect of the invention, DNA fragments included or comprised within the sequences, the access numbers of which are shown in table 2 below, are amplified.

According to a more specific embodiment, the amplified regions have a size of between 99 and 686 nucleotides and contain variable regions used for identification. According to an even more specific embodiment, the variable regions contain or are included within sequences SEQ ID NO:61 to SEQ ID NO:76 or complementary sequences, the positions of which are shown in table 2.

According to another embodiment, the amplification products that allow different bacterial species and groups to be identified are detected by means of probes. According to a more preferred embodiment, said probes have a length of between 15 and 25 nucleotides. And, according to an even more preferred embodiment, the probes have sequences SEQ ID NO:9-24 or complementary sequences (Table 2).

The primers can be designed by means of multiple alignment using computer programs such as CLUSTAL X, which allows the identification of highly conserved regions that act as moulds. According to another specific embodiment, the primers hybridize the genes indicated in table 2 below and particularly those with sequences with the access numbers shown in table 2 below. According to an even more specific embodiment, the primers have sequences SEQ ID NO:7-8 or complementary sequences.

### Brief explanation of the tables:

➢ Column 1 (organism) indicates the bacterial species or group of bacterial species detected in each case.
➢ Column 2 (gene) indicates the gene or genome region used to detect the bacterial species or group of species in column 1.
➢ Column 3 (primer) indicates the sequence of the pair of primers required to carry out the amplification of variable gene regions or genome regions indicated in each table (column 2).
➢ Column 4 (probe) indicates the sequence of the probes used to detect the bacterial species or group of species referenced in column 1 of each table.
➢ Column 5 (sequence 5'-3') indicates the sequence references of the variable regions which are amplified to detect each bacterial species or group of species.
➢ Column 6 (position 5'-3'):
   ∘ The first row: indicates a sequence code relative to a gene or genome region referenced in column 2, in addition to the specific position of said sequence in which the primer is hybridized (column 3).
   ∘ The second to the last row of each table indicate a sequence code relative to a gene or genome region referenced in column 2, in addition to the specific position of said sequence to which the probe is joined (column 4).

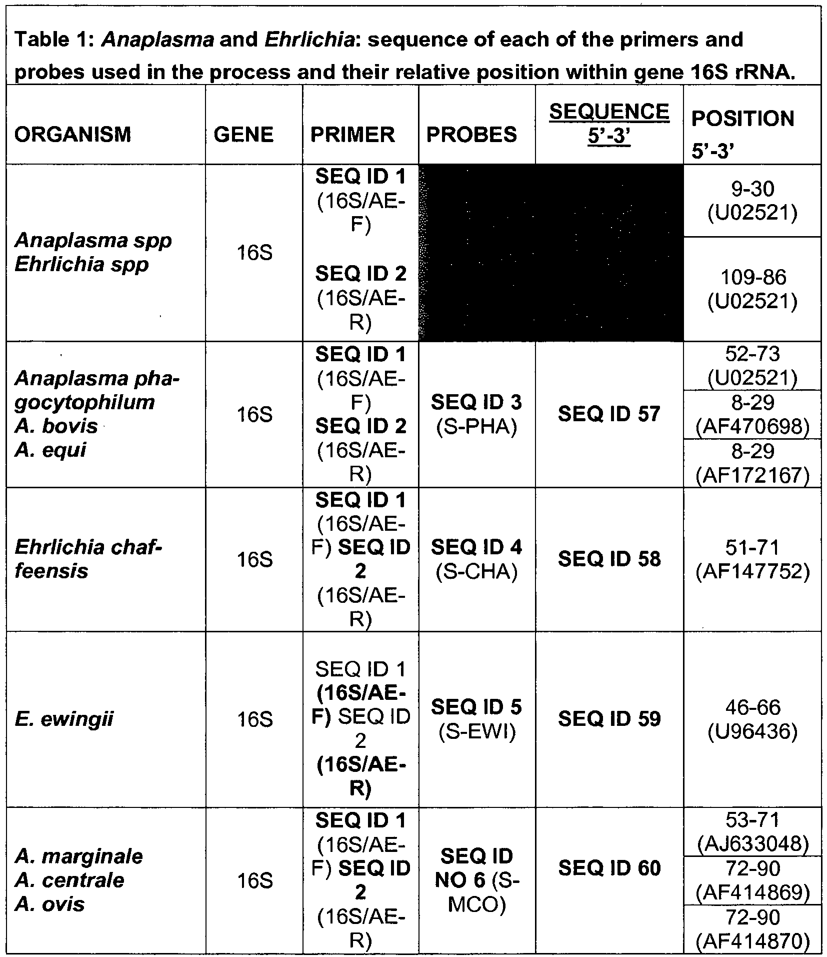

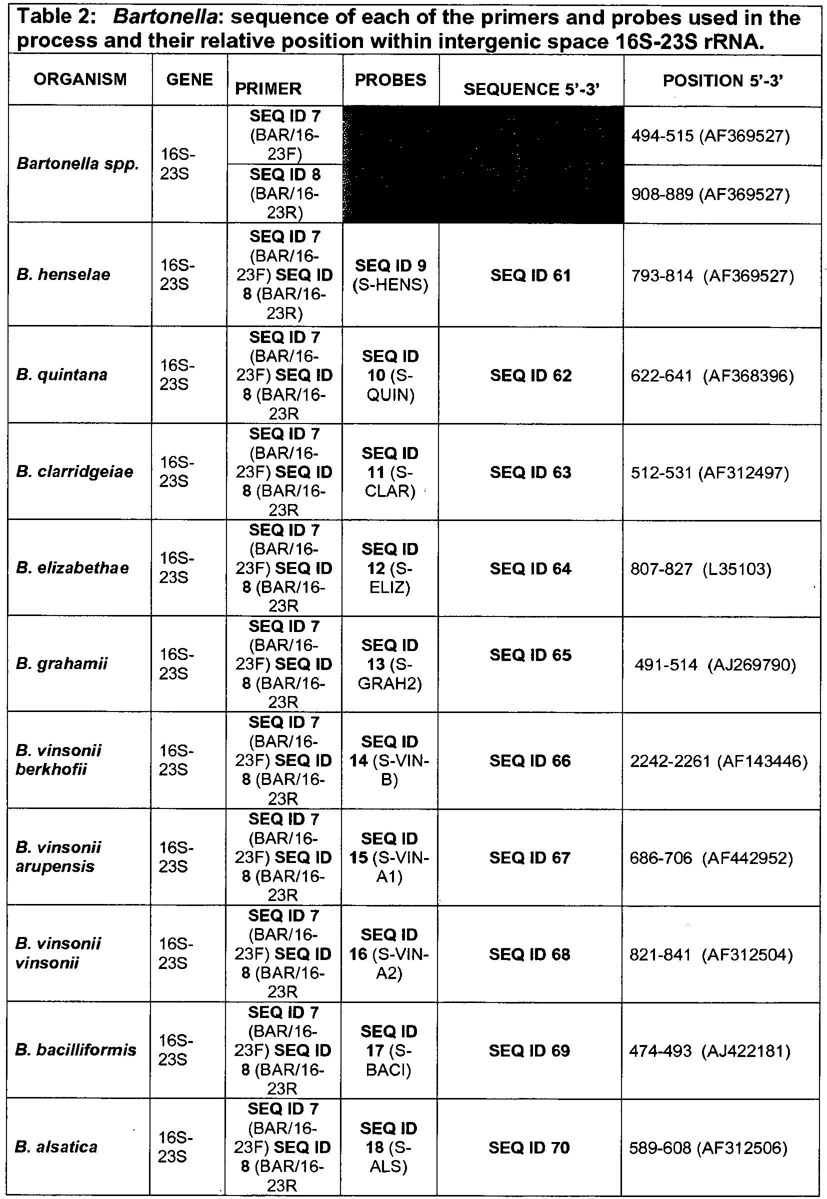

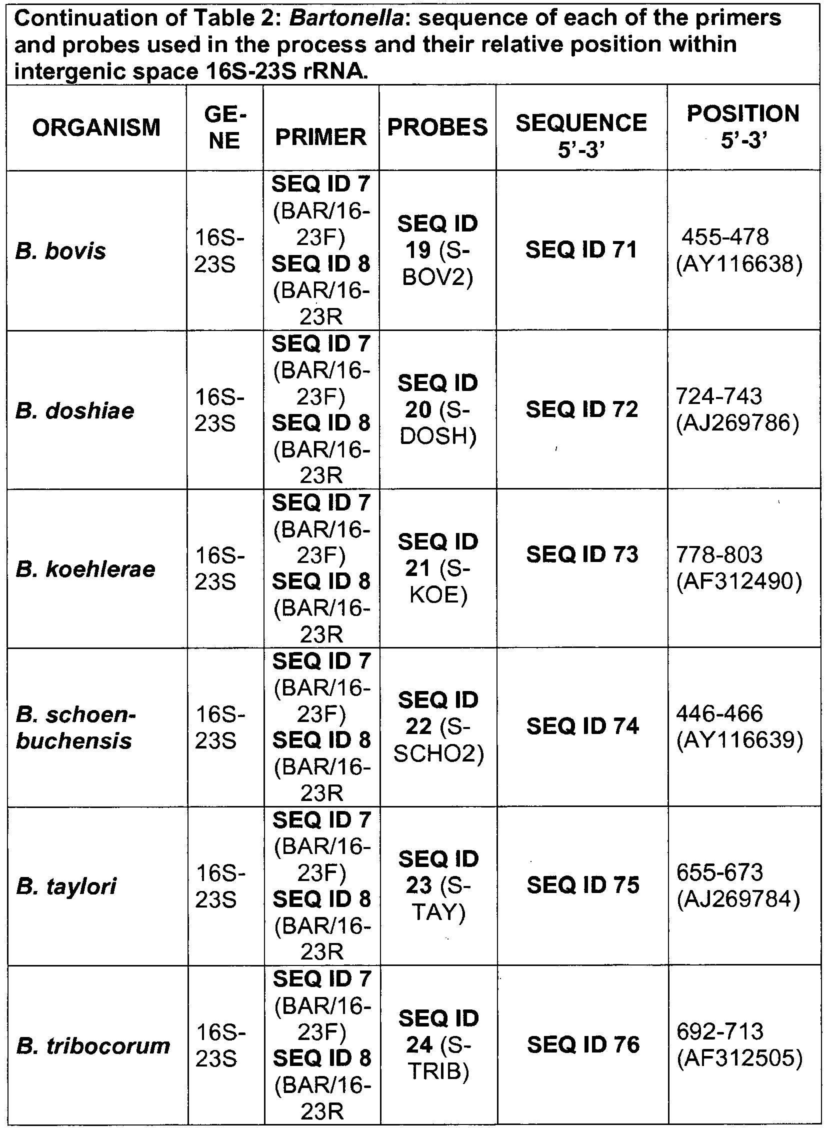

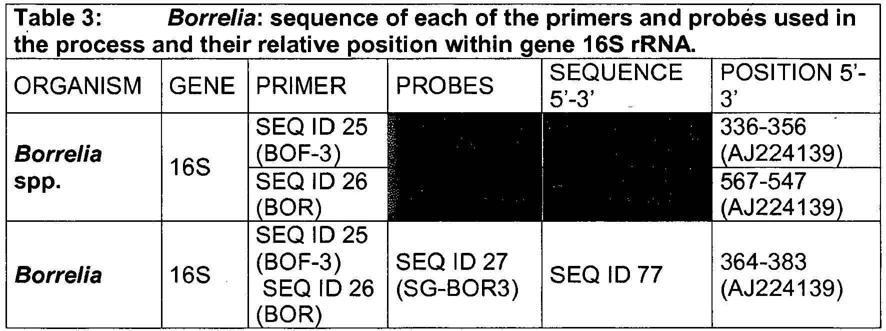

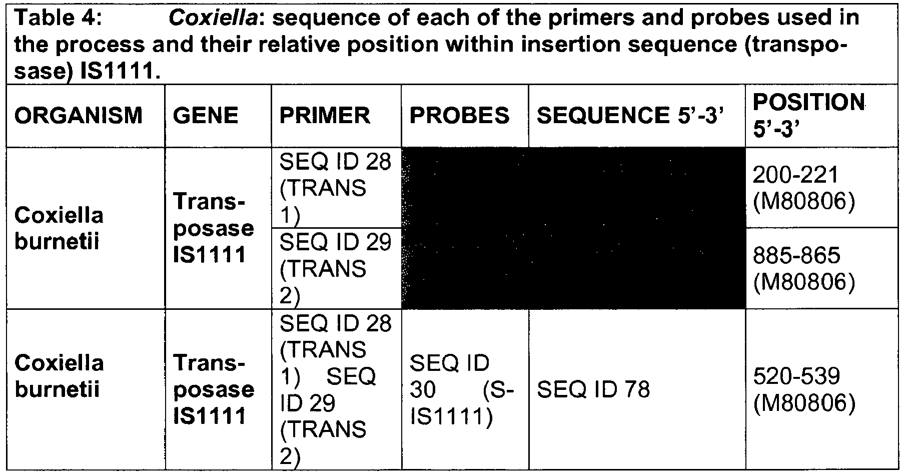

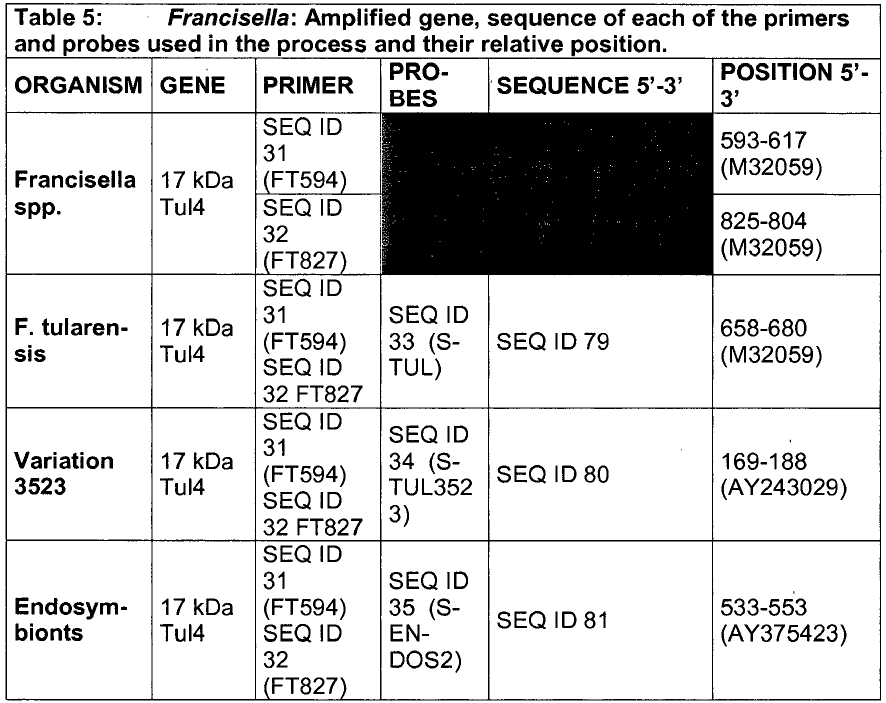

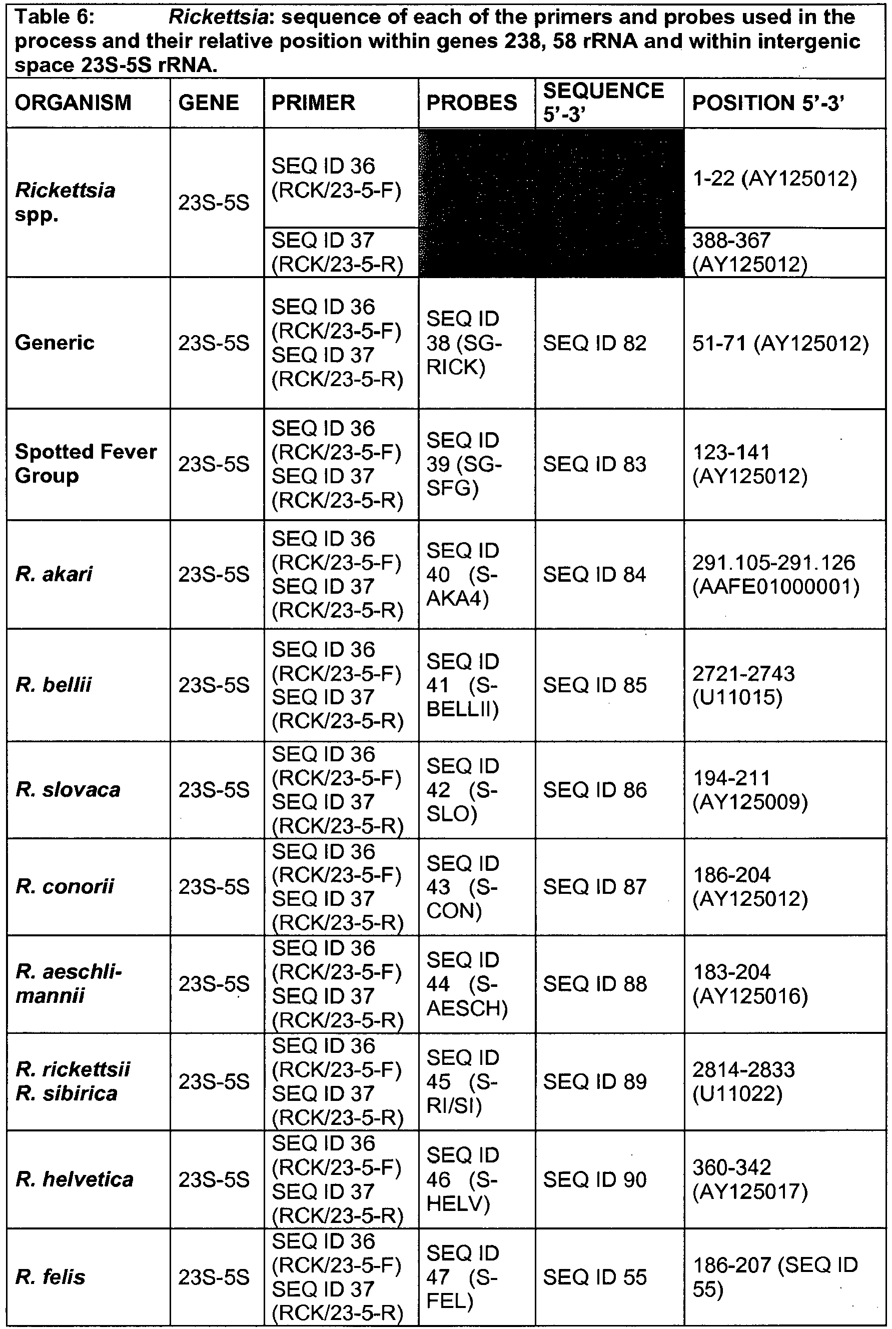

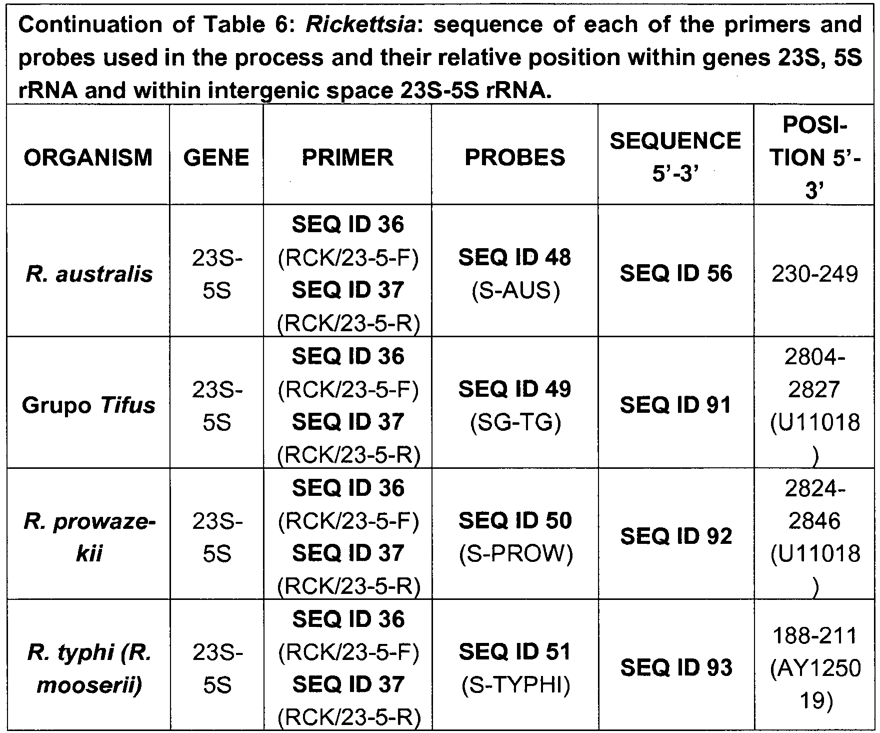

Given the abundance of PCR inhibitors, such as humic and fulvic acid, heavy metals, heparin, etc. which can produce false negatives and, despite the methods that exist to reduce the concentration of this type of molecules, we recommend (cf. J. Hoorfar et al., "Making internal Amplification control mandatory for diagnostic PCR" J. of Clinical Microbiology, Dec. 2003, pp. 5835) that the PCR tests contain an Internal Amplification Control (IAC). Said IAC is no more than a DNA fragment which is amplified simultaneously with the target sample, in such a way that its absence at the end of the testing process indicates the presence of factors which have caused unwanted development of the PCR.

A second method similar to the previously described, including at least one IAC, preferably comprised of a DNA sequence of the **Tetrahydrocannabinol Synthase** gene of the ***Cannabis sativa*** species and, more preferably, of a sequence with access number AB183705.

According to a more preferred embodiment, a region of the AB183705 sequence is amplified, said sequence being included within SEQ ID NO: 94 or complementary sequences (Table 7).

According to another preferred embodiment, the region is amplified by means of specific primers, the sequences of which comprise or are included within SEQ ID NO: 52 and SEQ ID NO: 53 or complementary sequences.

Within the context of this description, the term *"specific"* implies that the primers comprise a nucleotide sequence fully complementary to the

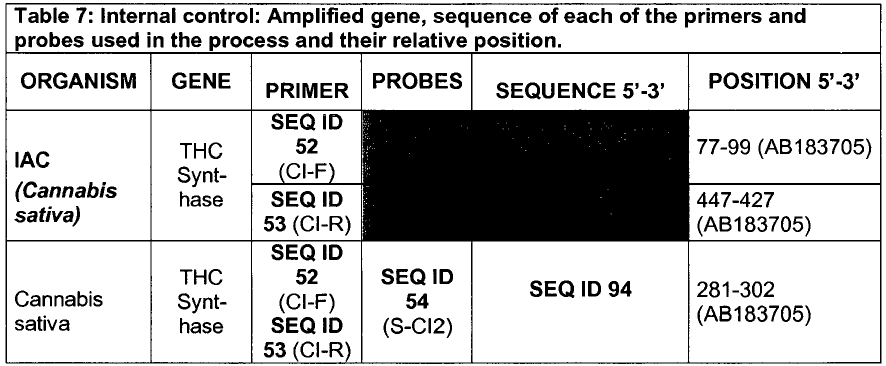

genes or genic fragments used by the present invention.

The term *"variable regions"* refer to DNA sequences which allow the identification of the bacterial species and groups identified by the present invention.

According to another embodiment, IAC amplification is detected by means of hybridization with probes. According to a more preferred embodiment, said probes have a length of 15 to 25 nucleotides. And, in an even more preferred embodiment, said probes have a sequence comprised or included in SEQ ID NO: 54 or complementary sequences.

The method disclosed herewith allows the detection of the aforementioned bacteria, independent of sample origin. Said samples could have been obtained from biopsies, scrapings, insects, biological fluids (blood, urine, saliva, etc.), field, etc. Once taken, the sample is pretreated in order to carry out Multiple PCR and subsequent amplicon identification.

The invention also provides diagnosis kits to apply the method described by the invention, which contain:
➢ Specific primers with sequences: SEQ ID 7-8 and optionally SEQ ID 52 y 53 as IAC.
➢ Probes with sequences: SEQ ID 9-24 and optionally SEQ ID 54 (S-Cl2) as IAC.

In the same way, said kits can include all the reactive agents required to apply any of the methods described. This includes, without any type of limitation, the use of buffers, polymerases, cofactors to optimize their activity, contamination-preventing agents, etc. On the other hand, the kits can include all of the supports and containers required for their startup and optimization.

The advantages of the present method and the kits with which to apply it include: speed (bacterial can be detected in less than 8 hours), specificity (the initiators used are specific to each species) and a high level of sensitivity.

Within the context of the specification description and claims, the word "comprises" and its variations, such as "comprising", does not intend to exclude other additives, components, constituent elements or stages. Both the embodiment example and complementary drawings do not intend to be limitative to the invention, but should rather be considered an aid to better understand it.

### DESCRIPTION OF THE DRAWINGS

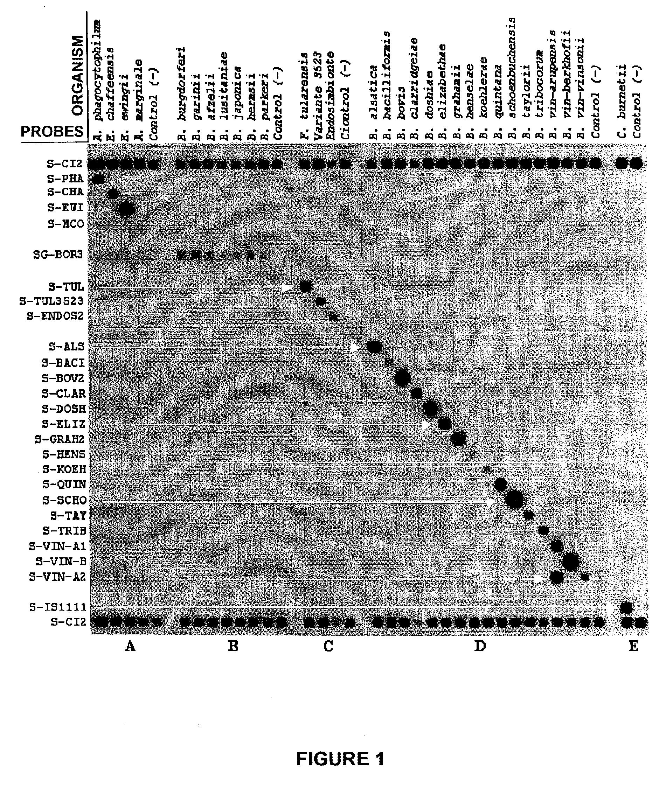

**Figure 1****.-** Hybridization membrane showing the validation of primers, probes and variable regions for the detection of *Anaplasma* and *Ehrlichia* (A), *Borrelia* (B), *Francisella* (C), *Bartonella* (D) and *Coxiella* (E) species, by means of specific probes (Tables 1-5). The S-Cl2 probe refers to the IAC probe (Table 7).

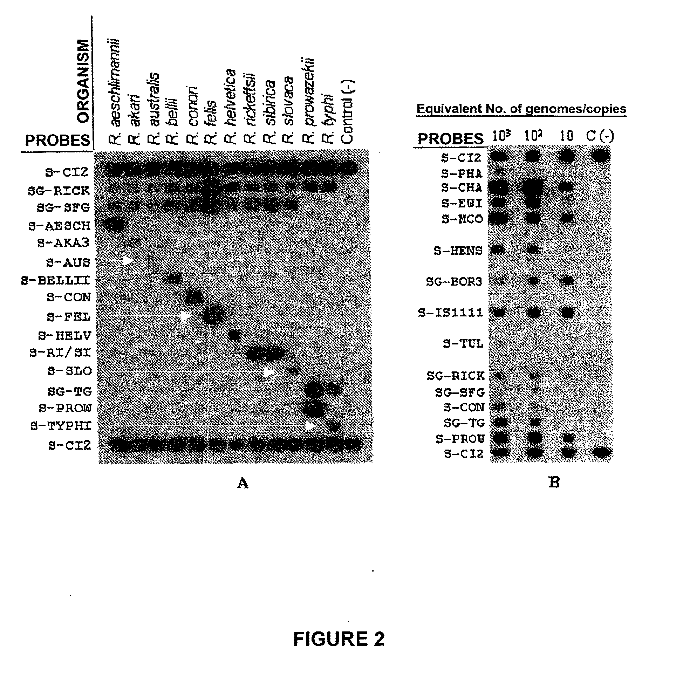

**Figure 2****.- A)** Hybridization membrane showing the validation of primers, probes and variable regions for the detection of *Rickettsia* species; B) Hybridization membrane showing an example of simultaneous detection of species belonging to the 7 genera. In this example: *A. phagocytophilum, A. marginale. E. chaffeensis, E. ewingi, B. henselae, B. burgdorferi, F. tularensis tularensis, R. conorii* and *R. prowazekii,* tested at 10³, 10² and 10 equivalent genome/copies. In both cases (A and B) the S-Cl2 probe, which refers to the IAC probe (Table 7), is used.

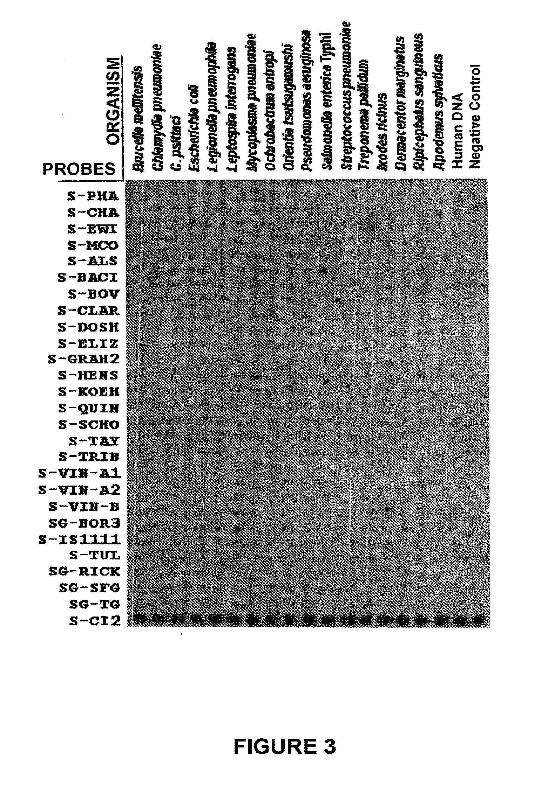

**Figure 3****.-** Hybridization membrane showing the results of a specificity study carried out within the indicated group of probes (Tables 1-7) on different species of bacteria, arthropods and mammals. The results reveal that the probes are not joined to the samples of the organisms tested in any case. The S-Cl2 probe refers to the IAC probe (Table 7).

### EMBODIMENT EXAMPLE

### Alignments, primer and probe designs

Conserved regions were identified by comparing and aligning multiple sequences obtained from public databases such as Genbank (http://www.ncbi.nlm.nih.gov), based on which specific primers were designed (Tables 1-6) for use in Multiple PCR. The compatibility between the primers, in addition to their optimal concentration, was empirically tested in the same manner as the magnesium salts and bovine seric albumin.

Variable regions were identified based on the selected sequence alignments, which allowed probes to be designed for the differentiation of bacterial species and genic groups (Tables 1-6) through BLB (Reverse Line Blotting) (kaufhold A, Podbielski A, Baumgarten G, Blokpoel M, Top J, Schouls L. Rapad Typing of group-a streptococci by the use of DNA amplification and nonradioactive allele-specific oligonucleotide probes. FEMS Microbiology Letters 119: 19-25 (1994)).

A first specificity analysis of each of the probes was carried out by comparing its sequence against public databases (Genbank) using computer programs such as BLAST (http://www.ncbi.hlm.nih.gov/blast/). Specificity was subsequently demonstrated by carrying out tests on a variety of DNA samples of different bacterial and eucaryotic species (Figure 3).

### DNA culture mediums and isolation

The species and genic groups selected for identification were obtained from private collections: the Spanish Type Culture Collection (CECT) and/or sample bank available at the National Microbiology Center (CNM). All of the species analyzed are shown in Table 8.

The isolation of genetic material was carried out using well-known procedures within the art and available on the market (DNA Mini Kit, Qiagen, N. Reference: 51304).

| **Table 8. Origin of DNA used in the invention** | | |
|---|---|---|
| **ORGANISM** | **NATIVE DNA (origln)** | **SYNTHETIC DNA** * |
| ***Anaplasma phagocytophilum*** | X (1) | |
| ***A. marginale*** | X (1) | |
| ***Ehrlichia chaffeensis*** | | X |
| ***E. ewingii*** | | X |
| ***Borrelia burgdorferi*** | X (2) | |
| ***B. garinii*** | X (2) | |
| ***B. afzelii*** | X (2) | |
| ***B. lusitaniae*** | X (2) | |
| ***B. japónica*** | X (2) | |
| ***B. hermsii*** | X (2) | |
| ***B. parkeri*** | X (2) | |
| ***Francisella tularensis tularensis*** | X (3) | |
| ***F. tularensis subesp. holarctica*** | X (3) | |
| ***F. tularensis subesp. Novicida*** | X (3) | |
| ***Francisella variante 3523*** | | X |
| ***Francisella Endosimbiontes*** | | X |
| ***Bartonella alsatica*** | X (4) | |
| ***B. bacilliformis*** | X (4) | |
| ***B. bovis*** | X (4) | |
| ***B. clarridgeiae*** | X (4) | |
| ***B. doshiae*** | X (4) | |
| ***B. elizabethae*** | X (4) | |
| ***B. grahamii*** | X (4) | |
| ***B. henselae*** | X (4) | |
| ***B. koehlerae*** | X (4) | |
| ***B. quintana*** | X (4) | |
| ***B. schoenbuchensis*** | X (4) | |
| ***B. taylorii*** | X (4) | |
| ***B. tribocorum*** | X (4) | |
| ***B. vinsonii subesp. Arupensis*** | X (4) | |
| ***B. vinsonii subesp. Berkhofii*** | X (4) | |
| ***B. vinsonii subesp. Vinsonii*** | X (4) | |
| ***Coxiella burnetii*** | X (5) | |
| ***Rickettsia aeschlimannii*** | | X |
| ***R. akari*** | X (5) | |
| ***R. australis*** | X (5) | |
| ***R*. *bellii*** | X (5) | |
| ***R. conori*** | X (5) | |
| ***R. felis*** | X (5) | |
| ***R. Helvetica*** | X (5) | |
| ***R. rickettsii*** | X (5) | |
| ***R. sibirica*** | | X |
| ***R*. *slovaca*** | X (5) | |
| ***R. prowazekii*** | | X |
| ***R. typhi*** | X (5) | |
| ***Brucella melitensis*** | X (6) | |
| ***Chlamydia pneumoniae*** | X (7) | |
| ***C. psittaci*** | X (7) | |
| ***Escherichia coli*** | X (6) | |
| ***Legionella pneumophila*** | X (8) | |
| ***Leptospira interrogans*** | X (4) | |
| ***Micoplasma pneumoniae*** | X (7) | |
| ***Ochrobactrum antropi*** | X (4) | |
| ***Orientia tsutsugamushi*** | X (5) | |
| ***Pseudomonas aeruginosa*** | X (4) | |
| ***Salmonella enterica Typhi*** | X (4) | |
| ***Streptococcus pneumoniae*** | X (6) | |
| ***Treponema pallidum*** | X (7) | |
| ***Ixodes ricinus*** | X (9) | |
| ***Dermacentor marginatus*** | X (9) | |
| ***Ripicephalus sanguineus*** | X (9) | |
| ***Apodemus sylvaticus*** | X (9) | |
| ***Human DNA*** | X (10) | |
| ***Internal Control*** | | X |

### Origin of natural DNA:

1: Positive sample.
2: Axenic culture medium, as described in:
   Benach JL, Coleman JL, and Golightly MG. 1988. A murine monoclonal antibody binds an antigenic determinant in outer surface protein A, an immunodominant basic protein of the Lyme disease spirochete. J. Immunol. 140:265-72.
3: Axenic culture medium, as described in:
   Anda P, Segura del Pozo J, Diaz Garcia JM, Escudero R, Garcia Peña FJ, Lopez Velasco MC, Sellek RE, Jimenez Chi-Ilaron MR, Sanchez Serrano LP, Martinez Navarro JF. 2001. Waterborne outbreak of tularemia associated with crayfish fishing. Emerg. Infect. Dis. 7 (Suppl):575-82.
4: Composition of axenic culture mediums specific to each species available at: http://cip.pasteur.fr/index.html.en
5: Propagation in cellular cultures using the "shell vial" technique, as described in:
   Marrero M, Raoult D. 1989. Centrifugation-shell vial technique for rapid detection of Mediterranean spotted fever rickettsia in blood culture. Am. J. Trop. Med. Hyg. 40: 197-9.
6: "Mueller Hinton" agar culture enriched with 5% of ram blood.
7: DNA extracted from slides for indirect commercial immunofluorescence.
8: Axenic culture medium, as described in:
   Edelstein PH. 1981. Improved semiselective medium for isolation of Legionella pneumophila from contaminated clinical and environmental samples. J. Clin. Microbiol. 14:298-303.
9: DNA extracted from pathogen-free specimens.
10: DNA extracted from clinical samples of patients with unrelated diseases.

### Synthetic DNA

Synthetic DNA was prepared according to the corresponding sequences listed in tables 1 to 7 (Column 6), by means of consecutive elongation of the DNA chain by PCR, using primers with approximately 70 nucleotides, of which approximately 20 nucleotides interoverlapped.

### Amplification, hybridization and validation

This step included the experimental analysis of the variable regions detected earlier using PCR for their validation. The isolated DNA was amplified using PCR (Saiki et al., (1985) Science 230, 1350; 1354), applying the following temperature cycle table and reaction mixture composition, together with the specific primers used previously for said purpose.

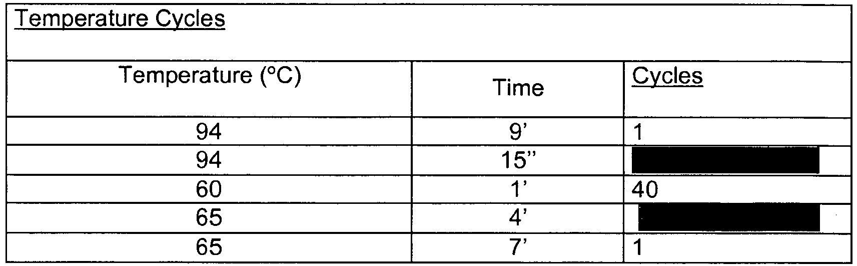

Reaction mixture composition for a final volume of 50 µL:
- H₂O: According to final DNA volume
- Buffer Taq Gold LD: 9 µL
- Cl₂Mg [3mM]: 6 µL
- dNTPs [200mM] : 1 µL x 4
- BSA [0,8ug/uL]: 4 µL
- 14 specific Primers (SEQ ID 1-2, SEQ ID 7-8, SEQ ID 25-26, SEQ ID 28-29, SEQ ID 31-32, SEQ ID 36-37, SEQ ID 52-53) [50 pm/µL]: 0,5 µL of each (7 µL)
- Taq Gold LD: 0.5 µL [2.5 units]
- Problem DNA: maximum 800 ng

The amplicons were sequenced for their validation, verifying that the amplified sequence coincided with the variable sequences inferred from bioinformatic studies. Subsequently, the amplicons were hybridized with specific probes according to the RLB protocol described by Sjoerd G. T. Rijpkema et al., Journal of Clinical Microbiology, Dec. 1995, p. 3091-3095, although applying the following modifications (Figures 1 and 2A):
- Substrate: Super Signal West Dura (Pierce, Ref: 34075)
- Probes: used with a concentration of between 0.2 and 3.2 picomoles/microlitre
- Incubation: at 55°C
- Wash: at 52°C

Hybridization results are shown in figures 1 and 2A, where we can observe that each of the probes of the invention become joined specifically to the amplicons of each of the bacterial species detected using the method of the invention.

### Preparation of samples and Multiple PCR

One of the advantages of using PCR and RLB analysis-based identification systems is that pure bacterial cultures are not required. In this manner and upon validation of the primers and probes using DNA samples of the different species and subspecies listed in tables 1-6, prepared following the procedures listed in table 8 and analyzed in duplicate, a Multiple PCR-based analysis of a DNA control mixture prepared under laboratory conditions was carried out, followed by the RLB test, using the specifically designed primers and probes and the previously indicated temperature cycles and reaction mixture composition, the results of which are shown in figure 2B. In said figure we can observe that it was possible to carry out the simultaneous detection of the bacterial species present in the sample.

### Detection of PCR inhibitors

An internal amplification control (IAC), which was amplified together with the target DNA, was created for the detection of PCR inhibitors, using specific primers (Table 7) designed according to the conserved regions of the AB183705 sequence (Table 7) belonging to the THC synthase gene of the *Cannabis sativa* species. Specifically, the IAC amplicon corresponds to a sequence of 371 pairs of bases, for which a probe was also designed (Table 7) for detection during RLB analysis.

### Specificity of the method

The high specificity of this method is based on the specificity of the primers and their probes, which were tested with another series of organisms (Table 9), following the method described by the present invention, verifying that the formation of amplicons detectable by means of hybridization (Figure 3) was not detected in any case.

| **Table 9: Specificity: unrelated species of bacteria, arthropods and mammals used in method development** | | |
|---|---|---|
| | ***SPECIES*** | **RLB RESULT** |
| | Bacteria | |
| 1 | *Brucella melitensis* | Negative |
| 2 | *Chlamydia penumoniae* | Negative |
| 3 | *C. psittaci* | Negative |
| | *Escherichia coli* | Negative |
| 4 | | |
| 5 | *Legionella pneumophila* | Negative |
| 6 | *Leptospira interrogans* | Negative |
| 7 | *Mycoplasma penumoniae* | Negative |
| 8 | *Ochrobactrum antropi* | Negative |
| 9 | *Orientia tsutsugamushi* | Negative |
| 10 | *Pseudomonas aeruginosa* | Negative |
| | *Salmonella enterica* Typhi | Negative |
| 11 | | |
| 12 | *Streptococcus pneumoniae* | Negative |
| 13 | *Treponema pallidum* | Negative |
| | | Negative |
| | Arthropods | |
| 14 | *Ixodes ricinus* | Negative |
| 15 | *Dermacentor marginatus* | Negative |
| 16 | *Ripicephalus sanguineus* | Negative |
| | | Negative |
| | Mammals | |
| 17 | *Apodemus sylvaticus* | Negative |
| 18 | Human | Negative |

## Claims

1. A pair of primers having a primer consisting of SEQ ID NO: 7 and a primer consisting of SEQ ID NO: 8, and capable of amplifying SEQ ID NO: 61-76 of bacterial species that cause zoonosis, belonging to the genus *Bartonella.*

2. Probe consisting of a sequence selected from SEQ ID NO: 9-24, and capable of hybridizing with SEQ ID NO: 61-76 of bacterial species that cause zoonosis, belonging to the genus *Bartonella.*

3. Kit comprising the pair of primers according to claim 1.

4. Kit according to claim 3, wherein it further comprises primers SEQ ID NO: 52 and SEQ ID NO: 53.

5. Kit comprising the probe according to claim 2.

6. Kit according to claim 5, wherein it further comprises probe SEQ ID NO: 54.

## Patentansprüche

1. Primerpaar mit einem Primer bestehend aus SEQ ID NO: 7 und einem Primer bestehend aus SEQ ID NO: 8, das in der Lage ist, die SEQ ID NO: 61-76 der Bakterienarten, die Zoonose verursachen und der Gattung *Bartonella* angehören, zu amplifizieren.

2. Sonde bestehend aus einer Sequenz, die aus SEQ ID NO. 9-24 ausgewählt wird und in der Lage ist, mit den SEQ ID NO: 61-76 der Bakterienarten, die Zoonose verursachen und der Gattung *Bartonella* angehören, zu hybridisieren.

3. Kit, der das Primerpaar gemäß Anspruch 1 umfasst.

4. Kit gemäß Anspruch 3, wobei er weiterhin die Primer SEQ ID NO: 52 und SEQ ID NO: 53 umfasst.

5. Kit, der die Sonde gemäß Anspruch 2 umfasst.

6. Kit gemäß Anspruch 5, wobei er weiterhin die Sonde SEQ ID NO: 54 umfasst.

## Revendications

1. Une paire d'amorces possédant une amorce consistant en SEQ ID NO:7 et une amorce consistant en SEQ ID NO:8, et capable d'amplifier SEQ ID NO:61-76 des espèces bactériennes qui provoquent des zoonoses, appartenant au genre *Bartonella.*

2. Sonde consistant en une séquence sélectionnée dans SEQ ID NO:9-24, et capable de s'hybrider avec SEQ ID NO:61-76 des espèces bactériennes qui provoquent des zoonoses, appartenant au genre *Bartonella.*

3. Kit comprenant la paire d'amorces selon la revendication 1.

4. Kit selon la revendication 3, dans lequel sont comprises en outre les amorces SEQ ID NO:52 et SEQ ID NO:53.

5. Kit comprenant la sonde selon la revendication 2.

6. Kit selon la revendication 5, dans lequel est comprise en outre la sonde SEQ ID NO:54.


### SEQUENCE LISTING

<110> Instituto de salud Carlos III
<120> PRIMERS, PROBES AND KITS FOR THE DETECTION OF BACTERIAL SPECIES BELONGING TO THE GENUS BARTONELLA
<130> 1613.1A
<150> ES20050001481
   <151> 2005-06-17
<160> 94
<170> PatentIn version 3.5
<210> 1
   <211> 22
   <212> DNA
   <213> Anaplasma spp
<400> 1
   cagaacgaac gctggcggca ag 22
<210> 2
   <211> 24
   <212> DNA
   <213> Anaplasma spp
<400> 2
   gcattactca cccgtctgcc actc 24
<210> 3
   <211> 22
   <212> DNA
   <213> Anaplasma bovis
<400> 3
   ggmttattct ttatagcttg ct 22
<210> 4
   <211> 21
   <212> DNA
   <213> Ehrlichia chaffeensis
<400> 4
   attgcttata accttttggt t 21
<210> 5
   <211> 21
   <212> DNA
   <213> Ehrlichia ewingii
<400> 5
   gaacaattcc taaatagtct c 21
<210> 6
   <211> 19
   <212> DNA
   <213> Anaplasma marginale
<400> 6
   cagcttgctg cgtgtatgg 19
<210> 7
   <211> 22
   <212> DNA
   <213> Bartonella spp
<400> 7
   ttgataagcg tgaggtcgga gg 22
<210> 8
   <211> 20
   <212> DNA
   <213> Bartonella henselae
<400> 8
   caaagcaggt gctctcccag 20
<210> 9
   <211> 22
   <212> DNA
   <213> Bartonella henselae
<400> 9
   atcggttcaa tcatatcgct tt 22
<210> 10
   <211> 20
   <212> DNA
   <213> Bartonella quintana
<400> 10
   cgcttatcca tttggtttaa 20
<210> 11
   <211> 20
   <212> DNA
   <213> Bartonella clarridgeiae
<400> 11
   acgatgctaa aagttgctat 20
<210> 12
   <211> 21
   <212> DNA
   <213> Bartonella elizabethae
<400> 12
   taagttccct tcaagaggat a 21
<210> 13
   <211> 24
   <212> DNA
   <213> Bartonella grahamii
<400> 13
   attcaagttg atgaatttgg ttat 24
<210> 14
   <211> 20
   <212> DNA
   <213> Bartonella vinsonii berkhofii
<400> 14
   tttcggacac tattgataaa 20
<210> 15
   <211> 21
   <212> DNA
   <213> Bartonella vinsonii arupensis
<400> 15
   acttgttgga attgcttaac c 21
<210> 16
   <211> 21
   <212> DNA
   <213> Bartonella vinsonii vinsonii
<400> 16
   atgaaaatat tgagagattt g 21
<210> 17
   <211> 20
   <212> DNA
   <213> Bartonella bacilliformis
<400> 17
   cctatgattg atttctaggc 20
<210> 18
   <211> 20
   <212> DNA
   <213> Bartonella alsatica
<400> 18
   gctggtgaaa cttgcttata 20
<210> 19
   <211> 24
   <212> DNA
   <213> Bartonella bovis
<400> 19
   cgttttgata gtcttttgtg ttgc 24
<210> 20
   <211> 20
   <212> DNA
   <213> Bartonella doshiae
<400> 20
   tttgaacctt ctctctttat 20
<210> 21
   <211> 27
   <212> DNA
   <213> Bartonella koehlerae
<400> 21
   ttaaattata tcactttggg tcatacg 27
<210> 22
   <211> 21
   <212> DNA
   <213> Bartonella schoenbuchensis
<400> 22
   gctgataagt ttgctgataa g 21
<210> 23
   <211> 19
   <212> DNA
   <213> Bartonella taylorii
<400> 23
   tatccatttc gcttaggca 19
<210> 24
   <211> 22
   <212> DNA
   <213> Bartonella tribocorum
<400> 24
   ttctattaag tttgtcaaag gg 22
<210> 25
   <211> 21
   <212> DNA
   <213> Borrelia spp
<400> 25
   taagaatctt ccgcaatggg c 21
<210> 26
   <211> 21
   <212> DNA
   <213> Borrelia spp
<400> 26
   atccgcctac tcacccttta c 21
<210> 27
   <211> 20
   <212> DNA
   <213> Borrelia spp
<400> 27
   tgacggagcg acactgcgtg 20
<210> 28
   <211> 22
   <212> DNA
   <213> Coxiella burnetii
<400> 28
   tatgtatcca ccgtagccag tc 22
<210> 29
   <211> 21
   <212> DNA
   <213> Coxiella burnetii
<400> 29
   cccaacaaca cctccttatt c 21
<210> 30
   <211> 20
   <212> DNA
   <213> Coxiella burnetii
<400> 30
   gcaagaatac ggactcacga 20
<210> 31
   <211> 23
   <212> DNA
   <213> Francisella spp
<400> 31
   gyaggtttag ckagctgttc tac 23
<210> 32
   <211> 23
   <212> DNA
   <213> Francisella tularensis
<400> 32
   ggagcytgcc attgtaatct tac 23
<210> 33
   <211> 23
   <212> DNA
   <213> Francisella tularensis
<400> 33
   agatactgct gctgctcaga cag 23
<210> 34
   <211> 20
   <212> DNA
   <213> Francisella tularensis
<400> 34
   gcatcagata agggcaccgc 20
<210> 35
   <211> 21
   <212> DNA
   <213> Francisella tularensis
<400> 35
   cagctacacc aacrgccgta g 21
<210> 36
   <211> 22
   <212> DNA
   <213> Rickettsia spp
<400> 36
   gataggtcrg rtgtggaagc ac 22
<210> 37
   <211> 22
   <212> DNA
   <213> Rickettsia spp
<400> 37
   tcgggayggg atcgtgtgtt tc 22
<210> 38
   <211> 21
   <212> DNA
   <213> Rickettsia spp
<400> 38
   tagctcgatt grtttacttt g 21
<210> 39
   <211> 19
   <212> DNA
   <213> Rickettsia spp
<400> 39
   actcacaarg ttatcaggt 19
<210> 40
   <211> 22
   <212> DNA
   <213> Rickettsia akari
<400> 40
   gatcatgcag caatacatta gc 22
<210> 41
   <211> 23
   <212> DNA
   <213> Rickettsia bellii
<400> 41
   gtgtttattc tataatatgt cag 23
<210> 42
   <211> 18
   <212> DNA
   <213> Rickettsia slovaca
<400> 42
   gtagcccctg ccacgata 18
<210> 43
   <211> 19
   <212> DNA
   <213> Rickettsia conorii
<400> 43
   gttatatact gtagccctg 19
<210> 44
   <211> 22
   <212> DNA
   <213> Rickettsia aeschlimanni
<400> 44
   atattatact gtatgtagcc cc 22
<210> 45
   <211> 20
   <212> DNA
   <213> Rickettsia rickettsii
<400> 45
   gttatactgt agtcctgcaa 20
<210> 46
   <211> 19
   <212> DNA
   <213> Rickettsia helvetica
<400> 46
   catggcttga tccacggta 19
<210> 47
   <211> 22
   <212> DNA
   <213> Rickettsia felis
<400> 47
   taatgttata ccgtggtccc gc 22
<210> 48
   <211> 20
   <212> DNA
   <213> Rickettsia australis
<400> 48
   gacaagttta gttatgcaat 20
<210> 49
   <211> 24
   <212> DNA
   <213> Rickettsia spp
<400> 49
   gttattctat cgttttatgt yacg 24
<210> 50
   <211> 23
   <212> DNA
   <213> Rickettsia prowazekii
<400> 50
   tacgatttga tagtaaagtt ttg 23
<210> 51
   <211> 24
   <212> DNA
   <213> Rickettsia typhi
<400> 51
   atgtcacgat ttgaccgtaa gatc 24
<210> 52
   <211> 23
   <212> DNA
   <213> Cannabis sativa
<400> 52
   atgatgctga gggtatgtcc tac 23
<210> 53
   <211> 21
   <212> DNA
   <213> Cannabis sativa
<400> 53
   gttttctcct ccaccaccac g 21
<210> 54
   <211> 22
   <212> DNA
   <213> Cannabis sativa
<400> 54
   gtggacactt tagtggagga gg 22
<210> 55
   <211> 377
   <212> DNA
   <213> Rickettsia felis
<220>
   <221> primer_bind
   <222> (186)..(207)
<400> 55
   gataggtcgg gtgtggaagc acagtaatgt gtgtagctaa ctgatactaa tagctcgatt 60
   gatttacttt gctgtgagat tacatatgca tatagtgtta attctataaa aatgtagtat 120
   caactcacaa agttatcagg ttaaattagc tttaccaatg aataaaaatg ttgttgcaca 180
   gctaataatg ttataccgtg gtcccgccac ggtatctaga aaaattttta atatttagat 240
   tcttgcttcc gcaggaatga taaatttagt catgcaacaa cattaacagc aaactataat 300
   acaaatctat ctttttaaaa gtttgtattg ctagcttggt ggttatagca tgagtgaaac 360
   acacgatccc atcccga 377
<210> 56
   <211> 343
   <212> DNA
   <213> Rickettsia australis
<220>
   <221> primer_bind
   <222> (230)..(249)
<400> 56
   ccgatactaa tagctcgatt gatttacttt gctgtgggat tacatatgca tatggtgtta      60
   attctataaa catgtaagtg tcaactcaca aagttatcag gttaaattag ctttatcaat     120
   gaataaagat gttgttgcac agctaataat gtcatctcgt ggcttgacca cagtatctag     180
   cagtatctag aaaaatttat aatatttgga ttcctgcttc cgtaggagtg acaagtttag     240
   ttatgcaata acattaacag cgaactataa tacaaatcta tttttttaaa agtttgtatt     300
   gctagcttgg tggttatagc atgagtgaaa cacacgatcc cga                       343
<210> 57
   <211> 22
   <212> DNA
   <213> Anaplasma bovis
<400> 57
   agcaagctat aaagaataak cc 22
<210> 58
   <211> 21
   <212> DNA
   <213> Ehrlichia chaffeensis
<400> 58
   aaccaaaagg ttataagcaa t 21
<210> 59
   <211> 21
   <212> DNA
   <213> Erlichia ewingii
<400> 59
   gagactattt aggaattgtt c 21
<210> 60
   <211> 19
   <212> DNA
   <213> Anaplasma marginale
<400> 60
   ccatacacgc agcaagctg 19
<210> 61
   <211> 22
   <212> DNA
   <213> Bartolella henselae
<400> 61
   aaagcgatat gattgaaccg at 22
<210> 62
   <211> 20
   <212> DNA
   <213> Bartonella quintana
<400> 62
   ttaaaccaaa tggataagcg 20
<210> 63
   <211> 20
   <212> DNA
   <213> Bartonella clarridgeiae
<400> 63
   atagcaactt ttagcatcgt 20
<210> 64
   <211> 21
   <212> DNA
   <213> Bartonella elizabethae
<400> 64
   tatcctcttg aagggaactt a 21
<210> 65
   <211> 24
   <212> DNA
   <213> Bartonella grahamii
<400> 65
   ataaccaaat tcatcaactt gaat 24
<210> 66
   <211> 20
   <212> DNA
   <213> Bartonella vinsonii berkhofii
<400> 66
   tttatcaata gtgtccgaaa 20
<210> 67
   <211> 21
   <212> DNA
   <213> Bartonella vinsonii arupensis
<400> 67
   ggttaagcaa ttccaacaag t 21
<210> 68
   <211> 21
   <212> DNA
   <213> Bartonella vinsonii vinsonii
<400> 68
   caaatctctc aatattttca t 21
<210> 69
   <211> 20
   <212> DNA
   <213> Bartonella bacilliformis
<400> 69
   gcctagaaat caatcatagg 20
<210> 70
   <211> 20
   <212> DNA
   <213> Bartonella alsatica
<400> 70
   tataagcaag tttcaccagc 20
<210> 71
   <211> 24
   <212> DNA
   <213> Bartonella bovis
<400> 71
   gcaacacaaa agactatcaa aacg 24
<210> 72
   <211> 20
   <212> DNA
   <213> Bartonella doshiae
<400> 72
   ataaagagag aaggttcaaa 20
<210> 73
   <211> 27
   <212> DNA
   <213> Bartonella koehlerae
<400> 73
   cgtatgaccc aaagtgatat aatttaa 27
<210> 74
   <211> 21
   <212> DNA
   <213> Bartonella schoenbuchensis
<400> 74
   cttatcagca aacttatcag c 21
<210> 75
   <211> 19
   <212> DNA
   <213> Bartonella taylori
<400> 75
   tgcctaagcg aaatggata 19
<210> 76
   <211> 22
   <212> DNA
   <213> Bartonella tribocorum
<400> 76
   ccctttgaca aacttaatag aa 22
<210> 77
   <211> 20
   <212> DNA
   <213> Borrelia burgdorferi
<400> 77
   cacgcagtgt cgctccgtca 20
<210> 78
   <211> 20
   <212> DNA
   <213> Coxiella burnetii
<400> 78
   tcgtgagtcc gtattcttgc 20
<210> 79
   <211> 23
   <212> DNA
   <213> Francisella tularensis
<400> 79
   ctgtctgagc agcagcagta tct 23
<210> 80
   <211> 20
   <212> DNA
   <213> Francisella tularensis
<400> 80
   gcggtgccct tatctgatgc 20
<210> 81
   <211> 21
   <212> DNA
   <213> Francisiella spp
<400> 81
   ctacggcygt tggtgtagct g 21
<210> 82
   <211> 21
   <212> DNA
   <213> Rickettsia spp
<400> 82
   caaagtaaay caatcgagct a 21
<210> 83
   <211> 19
   <212> DNA
   <213> Rickettsia spp
<400> 83
   acctgataac yttgtgagt 19
<210> 84
   <211> 22
   <212> DNA
   <213> Rickettsia akari
<400> 84
   gctaatgtat tgctgcatga tc 22
<210> 85
   <211> 23
   <212> DNA
   <213> Rickettsia bellii
<400> 85
   ctgacatatt atagaataaa cac 23
<210> 86
   <211> 18
   <212> DNA
   <213> Rickettsia slovaca
<400> 86
   tatcgtggca ggggctac 18
<210> 87
   <211> 19
   <212> DNA
   <213> Rickettsia conorii
<400> 87
   cagggctaca gtatataac 19
<210> 88
   <211> 22
   <212> DNA
   <213> Rickettsia aeschlimannii
<400> 88
   ggggctacat acagtataat at 22
<210> 89
   <211> 20
   <212> DNA
   <213> Rickettsia rickettsii
<400> 89
   ttgcaggact acagtataac 20
<210> 90
   <211> 19
   <212> DNA
   <213> Rickettsia helvetica
<400> 90
   taccgtggat caagccatg 19
<210> 91
   <211> 24
   <212> DNA
   <213> Rickettsia spp
<400> 91
   cgtracataa aacgatagaa taac 24
<210> 92
   <211> 23
   <212> DNA
   <213> Rickettsia prowazekii
<400> 92
   caaaacttta ctatcaaatc gta 23
<210> 93
   <211> 24
   <212> DNA
   <213> Rickettsia typhi
<400> 93
   gatcttacgg tcaaatcgtg acat 24
<210> 94
   <211> 22
   <212> DNA
   <213> Cannabis sativa
<400> 94
   cctcctccac taaagtgtcc ac 22